# EUROPEAN PATENT APPLICATION

(11) **EP 2 829 612 A1**
(43) Date of publication of application: **28.01.2015**
(21) Application number: 13177990.2
(22) Date of filing: 25.07.2013
(51) Int. Cl.: C12P 19/04, C12N 1/20, C12N 1/14

(54) **Method for producing exopolysaccharides, products and uses thereof**

(71) Applicant: Technische Universität München, 80333 München (DE)
(72) Inventor: Jakob, Frank, 85356 Freising (DE); Vogel, Rudi F., 85414 Kirchdorf (DE)
(74) Representative: Engelhard, Markus

(57) **Abstract**

The present invention relates to a method for producing exopolysaccharides (EPS), comprising pre-cultivating cells of a microorganism, that is capable of producing EPS, in a nutrient medium, separating the cells from the nutrient medium, re-suspending and incubating the cells in a solution comprising the inducer(s) for secretion of EPS forming enzymes, separating the cells from the solution, adding the supernatant to a second solution comprising the substrate(s) and optionally cofactor(s) for EPS formation and incubating, obtaining the EPS. The present invention further relates to an exopolysaccharide (EPS) obtained by the method and its uses in the food industry, pharmaceutics, cosmetic industry, biomedicine or environmental engineering, chemical industry or paper-, materials- or polymer industry. The present invention further relates to the enzymes obtained during the methods of the invention and their uses.

## Description

The present invention relates to a method for producing exopolysaccharides (EPS), comprising pre-cultivating cells of a microorganism, that is capable of producing EPS, in a nutrient medium, separating the cells from the nutrient medium, re-suspending and incubating the cells in a solution comprising the inducer(s) for secretion of EPS forming enzymes, separating the cells from the solution, adding the supernatant to a second solution comprising the substrate(s) and optionally cofactor(s) for EPS formation and incubating, obtaining the EPS. The present invention further relates to an exopolysaccharide (EPS) obtained by the method and its uses in the food industry, pharmaceutics, cosmetic industry, biomedicine or environmental engineering, chemical industry or paper-, materials- or polymer industry. The present invention further relates to the enzymes obtained during the methods of the invention and their uses.

### BACKGROUND OF THE INVENTION

Exopolysaccharides (EPS) are high-molecular-weight polymers that are composed of sugar residues and are secreted by many microorganisms into the surrounding environment. Microorganisms synthesize a wide spectrum of multifunctional polysaccharides including intracellular polysaccharides, capsular polysaccharides and extracellular polysaccharides (EPS). Exopolysaccharides generally consist of (modified) monosaccharides and, tentatively, some non-carbohydrate substituents, such as acetate, pyruvate, succinate, and phosphate. Owing to the wide diversity in composition, EPS have potential for multifarious applications in various food and pharmaceutical industries. Many microbial EPS provide properties that are as powerful and versatile as those of the gums currently in use.

Many EPS, such as glucans and fructans, are produced extracellularly by the activity of glycosyl- or fructosyltransferases, respectively, which use the disaccharide sucrose as main substrate and are secreted by microorganisms, such as bacteria and fungi, into the culture medium either constitutively or inducibly. These enzymes cleave sucrose in a first step, and afterwards transfer either glucose or fructose to a growing sugar polymer, forming glucans or fructans. The remaining free sugar monomers are subsequently metabolized. Therefore, glycosyl- or fructosyltransferase secreting microorganisms benefit from a protective biopolymer as well as from additional carbon sources for cell growth in the presence of sucrose. Both constitutive and inducible producers of EPS forming enzymes show consequently enhanced secretion rates of these enzymes in the presence of sucrose. Homopolysaccharides are generally produced in higher amounts than heteropolysaccharides, which consist of different sugar monomers and are synthesized in more energy-consuming biosynthetic pathways, and are therefore promising compounds for commercial applications due to their high production yields and functional properties (Korakli & Vogel, 2006). For commercial purposes, EPS are currently isolated from fermented fermentation broths, after separation of biomass, by e. g. alcoholic precipitation, being subsequently purified (Donot et al., 2012).

A vast number of microbial EPS have been reported over recent decades, and their composition, structure, biosynthesis and functional properties have been studied. Despite the great diversity of molecular structures described for bacterial EPS, only a few (such as dextrans, xanthans and gellans) have been industrially developed. The main constraints to full commercialization are their production costs, mostly related to substrate cost and downstream processing (Freitas et al., 2011; Naessens et al., 2005).

The microbial production of chemicals and enzymes is severely affected by fermentation conditions and downstream processing. The synthesis of viscous biopolymers additionally hinders the fermentation process, namely stirring and oxygenation, as well as the recovery of highly purified molecules. Microbial fermentations are coincident with continuous changes of extracellular conditions, due to the consumption of nutrients, accumulation of metabolites and thereby variations of pH, which have distinctive influences on the optimal production/secretion rates and activities of polysaccharide forming enzymes, which affect structure and functionality of the EPS. Such variations, which are hardly controllable due to an increasing viscosity of the fermentation broth, often negatively influence the amount and composition of the produced EPS. Due to the coercive use of complex nutrient media components during a microbial fermentation, these components must be separated from the produced EPS in cost- and time-consuming procedures.

A pure enzymatic synthesis of EPS is actually limited by the fact that the corresponding enzymes have to be purified extensively using chromatographic techniques. A complex purification process can furthermore unpredictably impair the optimal activity of the corresponding enzyme. Furthermore, EPS forming enzymes are considered as sole biocatalysts, while there is actually no evidence for potential further enzymes or secreted metabolites being involved in EPS synthesis. Theoretically, fermented supernatants of EPS forming microorganisms containing extracellular glucosyl- or fructosyltransferases could be used for EPS synthesis, while the produced EPS then again would have to be isolated from the media components by e. g. precipitation. Currently, an enzymatic EPS synthesis will be solely marketable, if these enzymes are recombinantly recovered from genetically modified organisms. This again limits the commercial applications of EPS, especially in the food industry.

US 4,879,228 describes the microbial production of polyfructose, namely a process for the production of a water-soluble levan by anaerobically fermenting a nutrient growth medium feedstock having a carbon source consisting essentially of a source of an assimilable sugar selected from the group consisting of sucrose, raffinose, and mixtures thereof with a microorganism capable of converting the sugar to levan and ethanol under nutrient growth conditions, wherein the microorganism is *Zymomonas mobilis* and the growth medium has initial sugar concentration of at least about 10% by weight and an initial concentration of an assimilable source of nitrogen no more than about half of that which would be optimal for the production of ethanol and wherein the fermentation is conducted at a temperature at least about 5°C below the optimum temperature for the production of ethanol, thereby increasing the amount of available fructose converted to levan.

US 6,855,524 B1 describes a process for producing exopolysaccharides by fermenting microorganisms that produce exopolysaccharides in a nutrient medium containing at least one carbon source assimilable by the microorganism and at least one organic nitrogen source, said nitrogen source being derived from a fraction of carob seed. The processes are described for the microorganisms selected from the group consisting of bacterial species of the genus *Xanthomonas, Alcaligenes, Agrobacterium, Arthrobacter*, *Azotobacter, Pseudomonas, Corynebacterium*, fungi of the genus *Sclerotium*, *Aspergillus*, and yeasts of the genus *Hansenula.* In particular, US 6,855,524 B1 describes a process for the production of xanthan gum by using bacterial species of the genus *Xanthomonas.*

There is a need in the art for improved means and methods for producing exopolysaccharides, such as homoexopolysaccharides, which in particular result in high yields of EPS and result in EPS with high purity.

### SUMMARY OF THE INVENTION

According to the present invention this object is solved by a method for producing exopolysaccharide(s) (EPS), wherein said method comprises the steps of
(a) pre-cultivating cells of a microorganism, that is capable of producing EPS, in a nutrient medium,
(b) separating the cells from the nutrient medium, optionally washing the cells;
(c) re-suspending the cells from step (b) in a solution comprising the inducer(s) for secretion of EPS forming enzymes and, optionally, further inducing compound(s),
   and incubating the cells in the solution, whereby the cells excrete the enzyme(s) for EPS formation into the solution,
(d) separating the cells from the solution;
(e) adding the supernatant of (d) to a second solution comprising the substrate(s) for EPS formation and, optionally, further cofactor(s),
   and incubating, whereby the EPS is formed;
(f) obtaining the EPS.

According to the present invention this object is solved by an exopolysaccharide (EPS) obtained by the method according to the invention.

According to the present invention this object is solved by the use of an exopolysaccharide (EPS) obtained by the method according to the invention or of an exopolysaccharide (EPS) according to the invention in the food industry, pharmaceutics, cosmetic industry, biomedicine or environmental engineering, chemical industry, paper/materials/polymer industry.

According to the present invention this object is solved by an enzyme or enzymes obtained during and/or after step (c), after step (d) and/or during and/or after the incubation of step (e) of the method according to the invention.

According to the present invention this object is solved by the use of the enzyme(s) for producing EPS, or in the food industry, pharmaceutics, cosmetic industry, biomedicine or environmental engineering, chemical industry, paper/materials/polymer industry.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "several minutes up to 48 hours" should be interpreted to include not only the explicitly recited values of 1 min, 2 min, to 48 hours, but also include individual values and sub-ranges within the indicated range. Thus, included in this numerical range are individual values such as 1 min, 2 min, 3 min , ... 59 min, 1 hour, 1 hour and 1 min, 1 hour and 2 min, ... 1 hour and 59 min, 2 hours, 2 hours and 1 min, ... 2 hours and 59 min 3 hours, ... 47 hours and 59 min, 48 hours and sub-ranges such as from 2 to 3 hours, from 30 min to 5 hours, from 10 hours to 15 hours, from 5 min to 2 hours and from 24 hours to 48 hours, etc. As an illustration, a numerical range of "2 g/L to 1,000 g/L" should be interpreted to include not only the explicitly recited values of 2 g/L and 1,000 g/L, but also include individual values and sub-ranges within the indicated range. Thus, included in this numerical range are individual values such as 2, 3, 4, 5, 6, 7, 8, 9, 10... 997, 998, 999 and 1,000 g/L and sub-ranges such as from 2 to 100 g/L, from 10 to 50 g/L, from 10 250 g/L and from 5 to 100 g/L, etc. This same principle applies to ranges reciting only one numerical value. Furthermore, such an interpretation should apply regardless of the breadth of the range or the characteristics being described.

### Method for producing exopolysaccharide(s) (EPS)

As discussed above, the present invention provides a method for producing exopolysaccharide(s) (EPS).

Said method comprises the steps of
(a) pre-cultivating cells of a microorganism, that is capable of producing EPS, in a nutrient medium,
(b) separating the cells from the nutrient medium, optionally washing the cells;
(c) re-suspending the cells from step (b) in a solution comprising the inducer(s) for secretion of EPS forming enzymes and, optionally, further inducing compound(s),
   and incubating the cells in the solution, whereby the cells excrete the enzyme(s) for EPS formation into the solution,
(d) separating the cells from the solution;
(e) adding the supernatant of (d) to a second solution comprising the substrate(s) for EPS formation and, optionally, cofactor(s),
   and incubating, whereby the EPS is formed,
(f) obtaining the EPS.

In the context of the present invention, the term "exopolysaccharide" denotes the extracellular polysaccharides produced by microorganisms, such as bacteria or fungi.

Exopolysaccharides are polymers of high molecular weight that are composed of sugar residues and are secreted by a microorganism into the surrounding environment.

The exopolysaccharides of high molecular weight can be used in industrial applications for their thickening, viscosifying, emulsifying, stabilizing, entrapment and releasing properties in especially aqueous media.

Exopolysaccharides have high molecular weights, most often greater than 1 x 10⁶ g/mol (measured by e.g. gel permeation chromatography).

Exopolysaccharides generally consist of
- (modified) monosaccharides, e.g. they are formed of units of glucose, mannose, galactose, rhamnose, glucuronic acid, mannuronic acid, guluronic acid;
- and optionally some non-carbohydrate substituents, such as acetate, pyruvate, succinate, and phosphate or derivatives thereof.

Their particular structure and their properties are described, for example, in Donot et al. 2012.

Owing to the wide diversity in composition, exopolysaccharides have potential for multifarious applications in various food and pharmaceutical industries.

For example, fructans are considered linear polymers of fructose molecules (Jakob et al., 2013). Fructans with a short chain length are known as fructooligosaccharides, whereas those with longer chains, i. e. a degree of polymerization > 10, are typically termed fructans. Fructans are built up of fructose residues, normally with a terminal sucrose unit (i.e. a glucose-fructose disaccharide). The linkage position of the fructose residues determines the type of the fructan. Linkage normally occurs at one of the two primary hydroxyl groups (OH-1 or OH-6), and there are two basic types of simple fructan:
1-linked: such as in inulin, where the fructosyl residues are linked by β-2,1-linkages;
   and
6-linked: such as in levan, where the fructosyl residues are linked by β-2,6-linkages.

For example, a glucan is a polysaccharide of D-glucose monomers linked by glycosidic bonds. Analogously to the classification of fructans, glucooligosaccarides are short-chain glucans with a degree of polymerization < 10.

The following are examples of glucans, wherein the α- and β-linkage types and their distributions within the polymer clarify the type of glucan:
dextran (α-1,6-glucan with α-1,3, α-1,4, or α-1,2 -branches);
pullulan (linear α-1,4- and α-1,6-glucan);
curdlan (β-1,3-glucan);
mutan (α-1,3-glucan);
alternan (alternating α-1,6 and α-1,3-linked glucan);
reuteran (α-1,4-glucan);
cellulose (β-1,4-glucan);
β-glucans (β-1,3 or β-1,6-glucans) (such as scleroglucans, schizophyllans, zymosans).

Preferably, the exopolysaccharides (EPS) produced by the methods of the invention are homopolysaccharides, such as
fructan(s), e.g. levans and inulins,
or
glucan(s), e.g. dextrans, mutans, reuterans, alternans,
or mixtures thereof.

In *step (a)* of the method of the invention, cells of a microorganism, that is capable of producing EPS, is pre-cultivated in a nutrient medium.

"Microorganisms that are capable of producing EPS" can be bacteria or fungi. These microorganisms secrete EPS forming enzymes, such as glycosyl- or fructosyltransferases.

A large number of EPS, such as glucans and fructans, are produced extracellularly by the activity of glycosyl- or fructosyltransferases belonging to the glycoside hydrolase families 70 and 68, respectively, which use the disaccharide sucrose as main substrate and are secreted by microorganisms either constitutively or inducibly. Glycosyl- or fructosyltransferases cleave sucrose in a first step, and afterwards transfer either glucose or fructose to a growing sugar polymer, forming glucans or fructans. The remaining free sugar monomers are subsequently metabolized. Both constitutive and inducible producers of EPS forming enzymes show consequently enhanced secretion rates of these enzymes in the presence of sucrose. Microorganisms, which secrete glycosyl-/fructosyltransferases belonging to the glycoside hydrolase families 70 and 68, respectively, are mainly found in the domains bacteria and fungi (Korakli & Vogel, 2006).

The microorganism is selected or chosen depending on the desired EPS.

The nutrient medium of step (a) is a suitable fermentation medium, which depends on the selected microorganism.

It comprises or contains carbon and nitrogen sources, vitamins, trace elements and essential growth elements.

In one embodiment, where the microorganism is a constitutive producer of the enzyme(s) for EPS formation, the nutrient medium in step (a) does preferably not contain the inducer(s) for secretion of EPS forming enzymes.

In one embodiment, where the microorganism is an inducible producer of the enzyme(s) for EPS formation, the nutrient medium in step (a) preferably contains the inducer(s) for secretion of EPS forming enzymes.

Pre-cultivation conditions, such as incubation temperature, nutrient composition of the fermentation medium, aerobic or anaerobic incubation, are preferably adapted to the typical growth requirements of the selected microorganism.

In a preferred embodiment, the cells are pre-cultivated in step (a) to the exponential growth phase.

In one embodiment, in step (a) the cells are pre-cultivated to the stationary growth phase.

The microorganism is preferably selected for its ability to effectively secrete the corresponding glycosyl- or fructosyltransferases.
Known suited microorganisms and fungi for the high level production of fructans are different species of acetic acid/lactic acid bacteria, species of the genera *Bacillus*, *Zymomonas*,, *Erwinia*, *Rahnella*, *Paenibacillus*, *Actinomyces*, *Aspergillus*, *Rhodotorula* (Velazquez-Hernandez et al., 2009).
Glucans (such as dextrans, alternans, reuterans, mutans) are produced in high amounts by many species of lactic acid bacteria (Korakli & Vogel, 2006) and some acetic acid bacteria (dextrans; Naessens et al., 2005). Cellulose production is widely spread among acetic acid bacteria (Tanaka et al., 2000).

In one embodiment, the microorganism is a genetically engineered strain that recombinantly produces EPS forming enzymes. Suitable recombinant expression strains, such as *Escherichia coli*, can inducibly (by addition of the inducer arabinose) produce fructosyltransferases originating from *Gluconobacter* species and can spontaneously secrete these enzymes into the extracellular environment (Jakob et al., 2012).

The microorganism, that is capable of producing EPS, is preferably selected from bacteria of the genus *Gluconobacter* (such as *Gluconobacter sp*.) or bacteria of the genus *Leuconostoc* (such as *Leuconostoc mesenteroides*).

For example, the water kefir isolate *Gluconobacter sp.* TMW 2.1191 is a constitutive levansucrase producer (Gulitz et al., 2011; Jakob et al., 2012) and can be used according to the invention to produce fructan. In this embodiment, the nutrient medium in step (a) does preferably not contain the inducer(s) for secretion of EPS forming enzymes.

For example, the water kefir isolate *Leuconostoc mesenteroides* TMW 2.1073 is an inducible dextransucrase producer, wherein the inducer is sucrose and optionally Ca²⁺ (Gulitz et al., 2011; Naessens et al., 2005; Robyt & Walseth, 1979) and can be used according to the invention to produce glucan (such as dextran). In this embodiment, the nutrient medium in step (a) preferably contains the inducer(s) for secretion of EPS forming enzymes, in particular sucrose and optionally Ca²⁺.

In *step (b)* of the method of the invention, the cells are separated from the nutrient medium.

Cells are separated by a suitable technique, e. g. centrifugation or filtration.

Optionally, the cells are washed, such as by re-suspension in a suitable solution (such as the solution used in step (c)) and then separated again, such as by centrifugation.

In *step (c)* of the method of the invention, the cells from step (b) are re-suspended.

According to the invention, the cells are re-suspended in a suitable solution that comprises the inducer(s) for secretion of EPS forming enzymes.

The solution is not a fermentation broth or nutrient medium.

The inducer(s) depend on the selected microorganism and the EPS to be produced.

Preferably, the inducer(s) for secretion of EPS forming enzymes is(are) selected from glucose, sucrose, fructose, galactose, trehalose, mannose, melobiose, raffinose, maltotriose, maltotetraose, maltose, lactose, lactulose, methyl-β-galactopyranoside, methyl-α-galactopyranoside, cellobiose, gentobiose, methyl-β-D-glucopyranoside, methyl-α-D-glucopyranoside, esculin, ribose, arabinose, xylose, palatinose, rhamnose, fucose, melezitose, D(+) arabitol, L(-) arabitol, xylitol, dulcitol, tagatose, glycerol, myoinositol, mannitol, maltitol, turanose, sorbitol, adonitol, lyxose, erythritol, hydrolyzed starch, starch hydrolyzates, mixtures and combinations of these sugars, and mixtures comprising at least one of these sugars.

In one embodiment, the inducer is sucrose.

The inducer(s) for secretion of EPS forming enzymes can also be EPS, such as glucooligosaccharides, glucans, fructooligosaccharides, fructans.

Thereby, the EPS utilized as inducer can be the same EPS (i.e. the same compound(s)/substance(s)) as the EPS to be produced or can be different to the EPS to be produced (i.e. different compound(s)/substance(s)).

Preferred inducer(s) are
sucrose, glucose, fructose, maltose, maltotetraose or
glucooligosacharides, glucans, fructooligosaccharides and/or fructans.

The solution in step (c) can comprise (further) inducing compounds, such as
- divalent cations
   that enhance the secretion process and stabilize the enzymes
   for example, Ca²⁺.

In one embodiment, the inducer is sucrose and the further inducing compound is Ca²⁺.

The solution in step (c) can furthermore comprise or contain salts, bases and acids, or combinations thereof for pH adjustment of the solution.

Preferably, the pH is selected in the range from around pH 3 to around pH 8.

Preferably, the solution in step (c) is prepared/composed/selected to obtain high secretion rates of active EPS forming enzymes.

After re-suspension, the cells are incubated in the solution, whereby the cells excrete the enzyme(s) for EPS formation into the solution.

The cells are incubated for a suitable time, such as for several minutes up to 48 hours, such as for 2 to 3 hours. The time can be varied according to the secretion rates of the producer strain.

The cells can be agitated, moved and/or stirred during incubation.

The cells can also be incubated without being moved.

The cells are incubated at a suitable temperature that depends on the microorganism selected.

In step (c), the pH of the solution can be kept constant during the incubation, such as due to the use of buffering substances.

For example, when using *Gluconobacter sp.,* the pH can be kept constant at around pH 5. For example, when using *Leuconostoc sp.,* the pH can be kept constant at around pH 5.

In *step (d)* of the method of the invention, the cells are separated from the solution.

The separation is preferably by centrifugation or filtration.

The cells are then discarded.

In *step (e)* of the method of the invention, the solution supernatant of step (d) is added to or mixed with a second solution.

The second solution is not a fermentation broth or nutrient medium.

According to the invention, the second solution comprises the substrate(s) for EPS formation and optionally cofactors.

The substrate(s) depend on the selected microorganism, their secreted enzymes for forming EPS and the EPS to be produced.

Preferably, the substrate(s) for EPS formation / EPS forming enzymes is(are) selected from glucose, sucrose, fructose, galactose, trehalose, mannose, melobiose, raffinose, maltotriose, maltotetraose, maltose, lactose, lactulose, methyl-β-galactopyranoside, methyl-α-galactopyranoside, cellobiose, gentobiose, methyl-β-D-glucopyranoside, methyl-α-D-glucopyranoside, esculin, ribose, arabinose, xylose, palatinose, rhamnose, fucose, melezitose, D(+) arabitol, L(-) arabitol, xylitol, dulcitol, tagatose, glycerol, myoinositol, mannitol, maltitol, turanose, sorbitol, adonitol, lyxose, erythritol, hydrolyzed starch, starch hydrolyzates, mixtures and combinations of these sugars, and mixtures comprising at least one of these sugars.

In one embodiment, the substrate is sucrose.

The substrate(s) for EPS formation / EPS forming enzymes can also be EPS, such as glucooligosaccharides, glucans, fructooligosaccharides, fructans.

Thereby, the EPS utilized as inducer can be the same EPS (i.e. the same compound(s)/substance(s)) as the EPS to be produced or can be different to the EPS to be produced (i.e. different compound(s)/substance(s)).

Preferred substrate(s) are
sucrose, glucose, fructose, maltose, maltotetraose or
glucooligosacharides, glucans, fructooligosaccharides and/or fructans.

The solution can comprise cofactors, such as the cofactor(s) of the EPS forming enzymes.

Cofactors can be:
- cations,
   that enhance the enzyme activity,
   for example, Ca²⁺.

In one embodiment, the substrate is sucrose and the cofactor is Ca²⁺.

The further inducing compound(s) of step (c) and the cofactor(s) of step (e) can be the same or different compound(s)/substance(s). Thus, these compound(s)/substance(s) can also be called "further inducing compound(s) and/or cofactor(s)".

The solution in step (e) can furthermore comprise or contain salts, bases and acids, or combinations thereof for pH adjustment of the solution.

After adding or mixing, the mixture of supernatant and the second solution is incubated such that the EPS is formed.

Preferably, the incubation is without stirring.

The incubation is carried out for a suitable time, such as for 24 hours. The incubation can be varied/extended according to the desired product yield.

The incubation is carried out at a suitable temperature, which depends on the corresponding enzyme's activity for EPS formation/production (glucan or fructan production).

Preferably, the solution in step (e) is prepared/composed/selected to obtain the desired composition and amount of the desired polymeric product, since the product yields and compositions of both glucans and fructans strongly depend on the used pH ranges and amounts of substrates/co-factors for EPS synthesis (Goldman et al., 2008; Koraldi & Vogel, 2006; Lakshmi Bhavani et al., 2010; Naessens et al., 2005)..

In step (e), the pH of the solution can be kept constant during the incubation, such as due to the use of buffering substances.

For example, when using *Gluconobacter sp.,* the pH can be kept constant at around pH 5. For example, when using *Leuconostoc sp.,* the pH can be kept constant at around pH 5.

Preferably, the pH is selected in the range from around pH 3 to around pH 8.

In one embodiment, the inducer used in the solution of step (c) and the substrate used in step (e) are the same compound (or one or several of the inducers used in step (c) and one or several of the substrates used in step (e) are the same compound).

For example, in the examples described herein:
- The water kefir isolate *Gluconobacter sp.* TMW 2.1191 utilizes sucrose as inducer for the secretion of the EPS forming enzyme levansucrase, which then needs sucrose as substrate for EPS formation (fructan).
- The water kefir isolate *Leuconostoc mesenteroides* TMW 2.1073 utilizes sucrose and optionally Ca²⁺ as inducers for the (production and) secretion of the EPS forming enzyme dextransucrase, which then needs sucrose and optionally Ca²⁺ as substrates for EPS formation (glucan/dextran).

In one embodiment, the solution in step (c) and the solution in step (e) is the same.

For example, in a case where the inducer(s) for secretion of EPS forming enzymes is(are) also the substrate(s) of the EPS forming enzyme, i.e. inducer(s) and substrate(s) are identical/the same compound(s).

In one embodiment, the EPS is directly isolated from the solution of step (c) axld/or step (e), in a case where the inducer(s) for secretion of EPS forming enzymes and the substrate(s) of the EPS fonning enzyme are identical.

The pH of the solution in step (c) aiid/or the solution in step (e) can be kept constant due to the use of buffering substances, such as around pH 5. The pH can also be varied such as if the pH for desired secretion rates in step (c) and the pH for desired product structure/composition/amount in step (e) are different.

For example, the dextran synthesizing activity of the dextransucrase of *Leuconostoc sp.* is reported to exhibit a maximum at pH 5.2 (i.e. at a pH around 5), which is considerably lower than that for the optimal production of the enzyme in culture (de Belder, 2003).

In one embodiment, the solution in step (c) and/or the solution step (e) is Na-acetate buffer (such as 0.1 M). The pH is around 5.0.

hi *step (f)* of the method of the invention, the EPS is obtained or isolated.

Preferably, the EPS is obtained or isolated in step (f) via
filtration,
(ultra)centrifugation,
ion exchange and/or
dialysis.

More preferably, obtaining the EPS in step (f) does not require (selective) precipitation.

In one embodiment, the EPS is isolated via dialysis, such as against water (e.g. ddH₂O).

According to the invention, the EPS is produced in a highly purified form, since it is produced in a solution (that comprises sugars, salts, acids and/or bases) and not in a fermentation broth. Thus, the obtaining or isolation can be carried out in a one-step procedure simply by filtration, (ultra)centrifugation, ion exchange and/or dialysis and requires no selective/alcoholic precipitation or removal of fermentation broth/medium components or cells.

This furthermore has the advantage that common technological problems known in the art, such as hindered stirring, oxygenation, cell separation and polymer purification, which are coincident with the increased thickening of the nutrient broth during microbial EPS production, do not occur using the present invention.

The method according to the invention can further comprise the step of stabilizing the obtained or isolated EPS of step (f).

Preferably, the stabilization is carried out via lyophilization or other suitable preparation methods.

For example, the stabilization is carried out via
- (conservative) dehydration, such as lyophilization;
   or
- drying, such as drying alone or after mixing with other components, entrapments and/or antioxidants.

According to the invention, the method can further comprise a heat deactivation of the residual proteins, such as enzymes.

This heat activation is preferably carried out after step (e) or (f), i.e. after the EPS is produced/obtained.

The yield of EPS which can preferably be produced with a method of the invention is in the range from about 2 g to about 1,000 g per litre cell culture, such as about 2 g to about 100 g per litre cell culture or about 10 g/L to about 50 g/L, such as about 20 g/L to about 40 g/L.

The obtained EPS yields depend on the selected microorganism, the secretion and conversion rates of its enzymes, the selected processing parameters, such as used amounts of solutions and used amounts of inducers, substrates, cofactors, and incubation times and temperatures.

In the exemplary embodiments described herein, 20 g fructan per litre cell culture or 35 g glucan per litre cell culture were obtained.

### EPS of the invention and its uses

As discussed above, the present invention provides an exopolysaccharide (EPS) obtained by the method according to the present invention.

The exopolysaccharide (EPS) is preferably a homopolysaccharide, such as a fructan, e.g. levan and inulin, or a glucan, e.g. dextran, mutan, reuteran, alternan, or mixtures thereof.

The EPS is preferably identified using common NMR spectroscopy techniques, such as e. g. ¹H/¹³C nuclear magnetic resonance (NMR) spectroscopy.

NMR spectra were recorded at Bruker Avance 300 MHz at 293K. The parameters used were previously described by Jakob et al. 2013.

In one embodiment which is described herein the fructan obtained has ¹³C NMR chemical shifts (ppm) of 62.72 (C1), 106.96 (C2), 79.10 (C3), 77.98 (C4), 83.05 (C5) and 66.15 (C6) and is, therefore, identified as levan (Jakob et al., 2013).

In one embodiment which is described herein the glucan obtained has ¹³C NMR chemical shifts (ppm) of 97.62 (C1), 71.32 (C2), 73.32 (C3), 69.44 (C4), 70.10 (C5) and 65.46 (C6) and is, therefore, identified as dextran. An additional main signal at 4.99 ppm located in the anomeric region of the ¹H NMR spectrum of this glucan, confirms the presence of dominating α-1,6-linkages (Shulda et al., 2011).

The EPS, preferably a fructan, obtained by the method according to the present invention is **characterized in that** it has a uniform particle size and a uniform molecular weight as detected by AF4-MALS-RI.

Wherein "AF4-MALS-RI" refers to asymmetric flow field-flow fractionation (AF4) coupled to a multi-angle laser light scattering (MALS) unit and a refractive index (RI) as quantitative detector.

Molecules were separated by AF4 (Eclipse, Wyatt Technology, Germany) and analyzed regarding their molecular weights and particles sizes by MALS (Dawn Heleos II, Wyatt Technology, Germany). Concentrations of molecules were determined using RI detection (Agilent Series 1200 G1362A, Agilent Technologies, Germany).

The methods used to separate and characterize fructan molecules were previously described by Jakob et al., 2013.

In one embodiment, the fructan obtained according to the invention has
- an average gyration radius R_{G} of 428 nm (as measured by AF4-MALS-RI);
- an average molecular weight M_{w} of 8.26 x 10⁹ Da (as measured by AF4-MALS-RI);
- a hydrodynamic coefficient value V_{G} of 0.28 (as measured by AF4-MALS-RI);
- a polydispersity index of 1.006 (as measured by AF4-MALS-RI).

The fructan particles obtained can be microscopically observed as uniform spherical particles of about 1µm in diameter.

As discussed above, the present invention provides the use of an exopolysaccharide (EPS) obtained by the method according to the invention or of an exopolysaccharide (EPS) according to the invention.

EPS are used in numerous industrial applications for their thickening, viscosifying, emulsifying, stabilizing properties, in especially aqueous media.

The EPS of the present invention / EPS obtained according to the invention are preferably used directly or in a e.g. product, formula or matrix.

The EPS of the present invention / EPS obtained according to the invention in the food industry, pharmaceutics, cosmetic industry, biomedicine or environmental engineering, chemical industry, paper-, materials- or polymer- industry

The EPS of the present invention / EPS obtained according to the invention are preferably used in
food industry,
   such as
   as thickening agent, stabilizing agent, emulsifying agent, gelating agent,
   as hydro-colloids,
   as inclusion bodies (e.g. for the controlled release of vitamins, aroma compounds)
pharmaceutics,
   such as for drug delivery, e.g. with spherical inclusion bodies,
   biological degradable synthetics
cosmetic industry
   such as
   as bioadhesive substances,
   as thickening agent, stabilizing agent, emulsifying agent, gelating agent, (e.g.
   in tooth paste, lipstick, creames)
biomedicine or environment engineering,
   such as
   in (artificial) biofilms
   blood plasma volume expanders
chemical industry
   such as
   as molecular weight standard
   as initiator molecules for further processing (derivatized polymers)
   as column materials for chromatographic techniques.
paper/materials/polymer industry
   such as
   as surface smoothener
   as structural improver
   as firmness improver
   as water binding agent
   as compatibility enhancer (e.g. in implants).

### Enzymes of the invention and its uses

As discussed above, the present invention provides an enzyme or enzymes obtained during a method according to the invention.

When carrying out the method of the invention, an enzyme or enzymes can be obtained in particular:
- during and/or after step (c),
- after step (d);
   and/or
- during and/or after the incubation of step (e).

An enzyme or enzymes are preferably glycosyl- and/or fructosyl-transferase(s) belonging to the glycoside hydrolase families 70 and 68, such as dextransucrase(s), alternansucrase(s), reuteransucrase(s), mutansucrase(s), levansucrase(s), inulosucrase(s). See also Korakli & Vogel, 2006.

The enzyme(s) can be obtained in form of a solution.

A solution obtained during and/or after step (c) contains a mixture of cells and enzymes. Such a solution can be used as starter solution for food fermentations, such as for the fermentation of dough, milk products, meat, beverages, vegetables or fruits. Such a solution can also be e.g. lyophilized and re-activated in a suitable fermentation medium or matrix for the above uses.

The enzyme(s) can also be obtained in form of a solution after step (d) and/or during and/or after the incubation of step (e).

Enzyme(s) can be recovered or obtained from these solutions, such as in powdered form, e.g. by lyophilisation.

The powdered form of enzyme(s) can be further used. For example, the enzyme(s) can be re-activated in a suitable medium.

The powdered form of a solution obtained during and/or after step (c) can be re-activated in a suitable fermentation medium or matrix.

The powdered form of a solution obtained after step (d) and/or during and/or after the incubation of step (e) can be re-activated in a suitable medium, preferably a suitable aqueous medium.

As discussed above, the present invention provides the use of an enzyme or enzymes obtained as described above.

The enzyme(s) are preferably used for producing EPS.

In one embodiment, the enzyme(s) are used in a method for producing EPS, such as in the method according to the invention.

In such an embodiment, the enzyme(s) either are used in form of solutions or re-activated from powdered form (as described above).

In such an embodiment, the solutions as described above for the method of the invention, which then contain the substrate(s) for EPS formation and, optionally, further cofactors and/or further component(s), as described above, are utilized. The solutions are added to and/or mixed with the enzyme(s) in order to allow EPS formation.

The enzyme(s) are preferably used in
food industry,
   such as
   for the enzymatic production
   of thickening agent, stabilizing agent, emulsifying agent, gelating agent, of hydro-colloids,
   of inclusion bodies (e.g. for the controlled release of vitamins, aroma compounds);
   as starter solution for food fermentations, such as for the fermentation of dough, milk products, meat, beverages, vegetables or fruits;
pharmaceutics,
   such as for the enzymatic production
   of drug delivery systems, e.g. with spherical inclusion bodies,
   of biological degradable synthetics
cosmetic industry
   such as for the enzymatic production
   of bioadhesive substances,
   of thickening agent, stabilizing agent, emulsifying agent, gelating agent, (e.g. in tooth paste, lipstick, creames)
biomedicine or environment engineering,
   such as for the enzymatic production
   of (artificial) biofilms
   of blood plasma volume expanders
chemical industry
   such as for the enzymatic production
   of molecular weight standard
   of initiator molecules for further processing (derivatized polymers)
   of column materials for chromatographic techniques.
paper/materials/polymer industry
   such as for the enzymatic production
   of surface smoothener
   of structural improver
   of firmness improver
   of water binding agent
   of compatibility enhancer (e.g. in implants).

The inventors have developed a method for the efficient and controlled production of microbial (homo)exopolysaccharides (preferably glucans and fructans), preferably derived from the substrate sucrose. The main principle of the present invention is to recover highly active, natively produced enzymes, which are effectively secreted into a solution (which solely comprises sugars, salts, acids and/or bases) by the selected microorganism. After cell separation, an up-scaled, enzymatically controlled EPS synthesis is directly initiated by adding the collected enzyme solution to a second defined solution system. Only small metabolites, such as residual sugars, salts, acids and/or bases, have to be separated from the produced EPS in a one-step procedure by e. g. dialysis, filtration, ion exchange or centrifugation. Potential residual enzymes can be heat deactivated. The purified EPS is preferably (freeze)-dried for stabilization.

According to the invention, the selected microorganism is pre-cultivated (preferably) to the exponential growth phase in a suitable fermentation medium with or without (constitutive the inducer for enzyme secretion. Cells are subsequently separated from the fermentation broth and (after an optional washing procedure) further incubated in a solution that contains the inducer for enzyme secretion for a suitable time. Afterwards, cells are separated from the solution and discarded. The remaining enzyme solution is finally mixed with a substrate-containing solution for large scale enzymatic conversions.

Thus, the present invention provides a method for producing EPS with the following advantages when compared to the known and commercially used methods for EPS production:
- EPS can be obtained in less time;
- natively produced, highly stabilized enzymes are recovered without needs of purification/modification;
- less materials are needed/consumed;
- higher yields of EPS are obtained;
- EPS with higher purity are obtained;
- EPS with higher uniformity are obtained;
- EPS with higher molecular weights can be obtained;
- EPS with tailor made properties are obtained;
- the method is easily reproducible and controllable.

Microbial synthesized glucans and fructans are widely used in biotechnological applications. The present invention is a biotechnological process for the production of such polysaccharides.

The following examples and drawings illustrate the present invention without, however, limiting the same thereto.

### BRIEF DESCRIPTION OF THE DRAWINGS

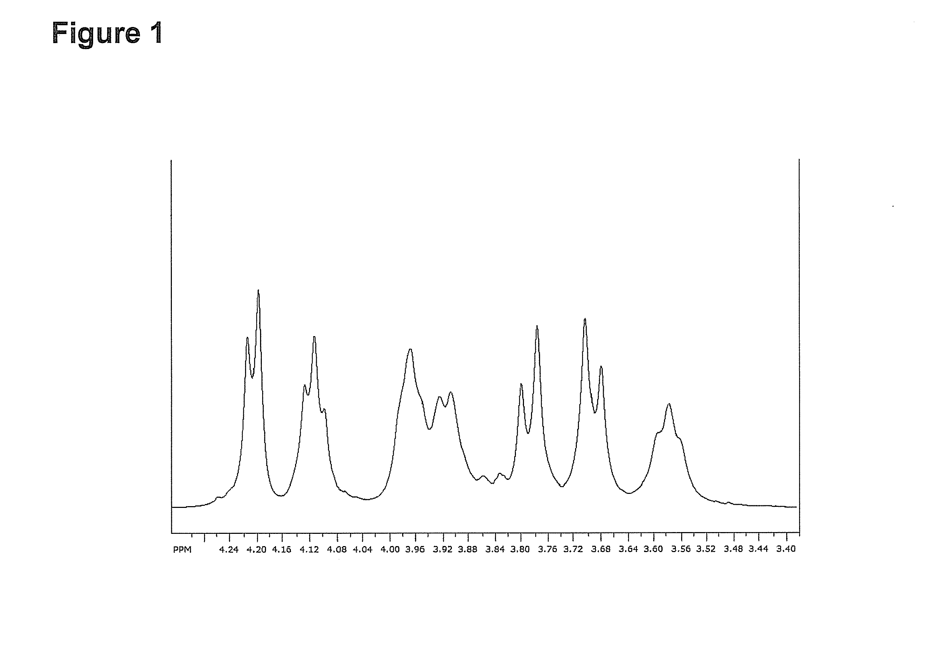

**Figure 1**: ¹H NMR spectrum of fructan produced by *Gluconobacter sp.* TMW 2.1191.
   The distributions of signals are identical with those of levans previously identified by Jakob et al., 2013.

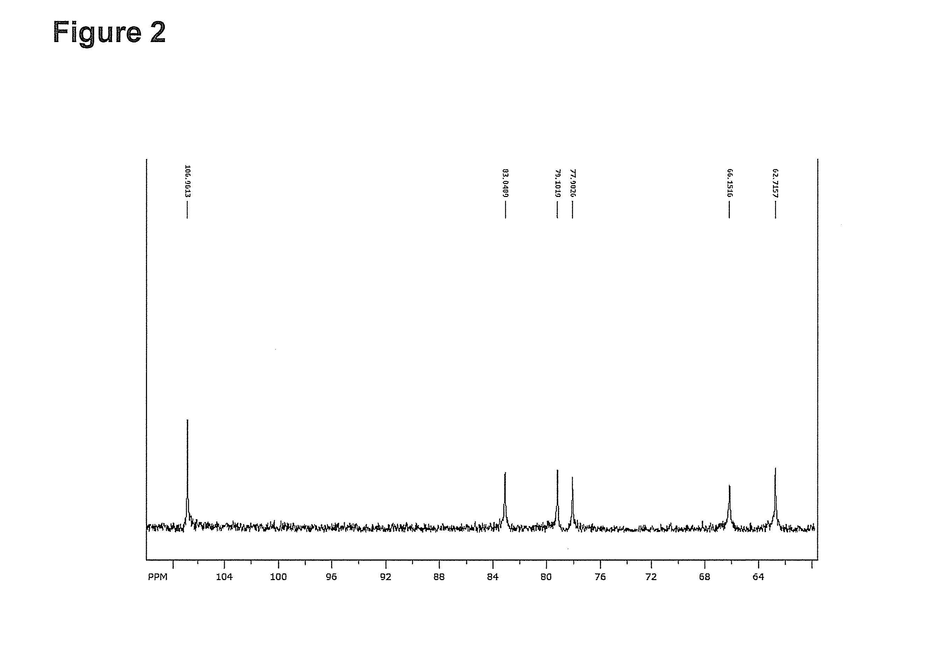

**Figure 2**: ¹³C NMR spectrum of fructan produced by *Gluconobacter sp.* TMW 2.1191.
   The ¹³C NMR chemical shifts (ppm) of the 6 main carbon signals are 62.72 (C1), 106.96 (C2), 79.10 (C3), 77.98 (C4), 83.05 (C5) and 66.15 (C6) and are characteristic for levans (Jakob et al., 2013).

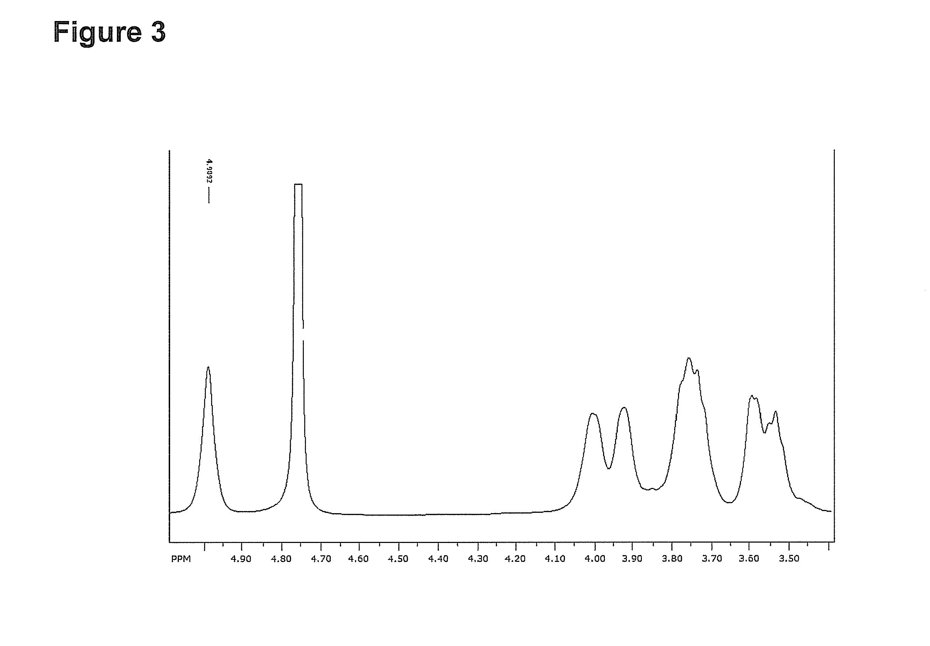

**Figure 3**: ¹H NMR spectrum of glucan produced by *Leuconostoc mesenteroides* TMW 2.1073.
   The signal at 4.99 ppm corresponds to the presence of a-1,6-lincages in dextrans (Shulda et al., 2011). The remaining signals are broadened, possibly due to the high molecular weight of this polymer. The large peak at 4.75 ppm corresponds to the HOD signal.

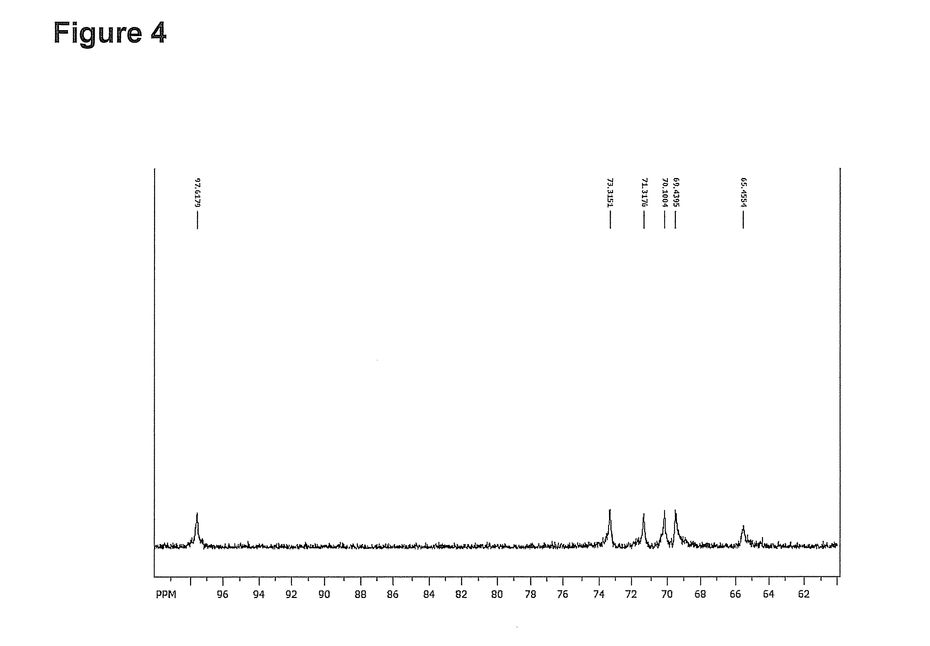

**Figure 4**: ¹³C NMR spectrum of glucan produced by *Leuconostoc mesenteroides* TMW 2.1073.
   The ¹³C NMR chemical shifts (ppm) of the 6 main carbon signals are 97.62 (C1), 71.32 (C2), 73.32 (C3), 69.44 (C4), 70.10 (C5) and 65.46 (C6) and are characteristic for dextrans (Shukla et al., 2011).

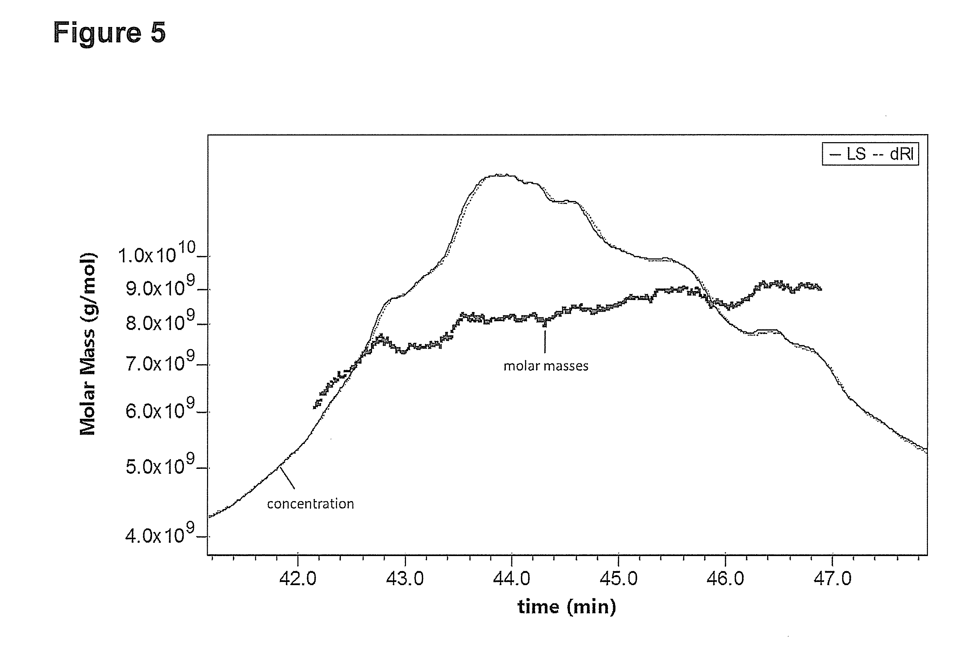

**Figure 5**: Elugram of fructan produced by *Gluconobacter sp.* TMW 2.1191 obtained from AF4-MALS-RI showing the relative concentration of levan molecules and their corresponding molar masses.

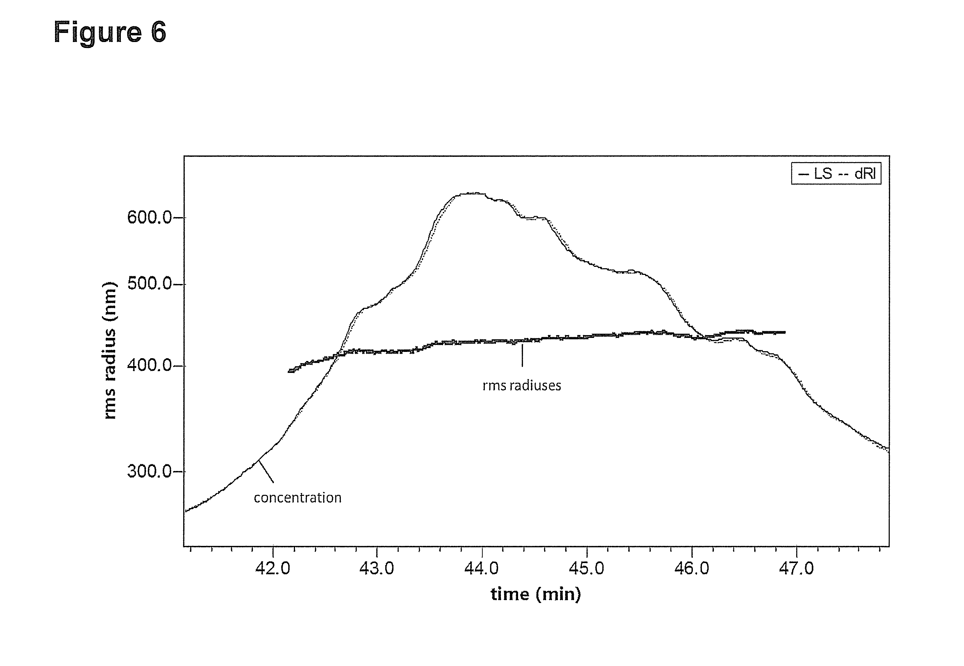

**Figure 6**: Elugram of fructan produced by *Gluconobacter sp.* TMW 2.1191 obtained from AF4-MALS-RI showing the relative concentration of levan molecules and their corresponding radiuses of gyration.

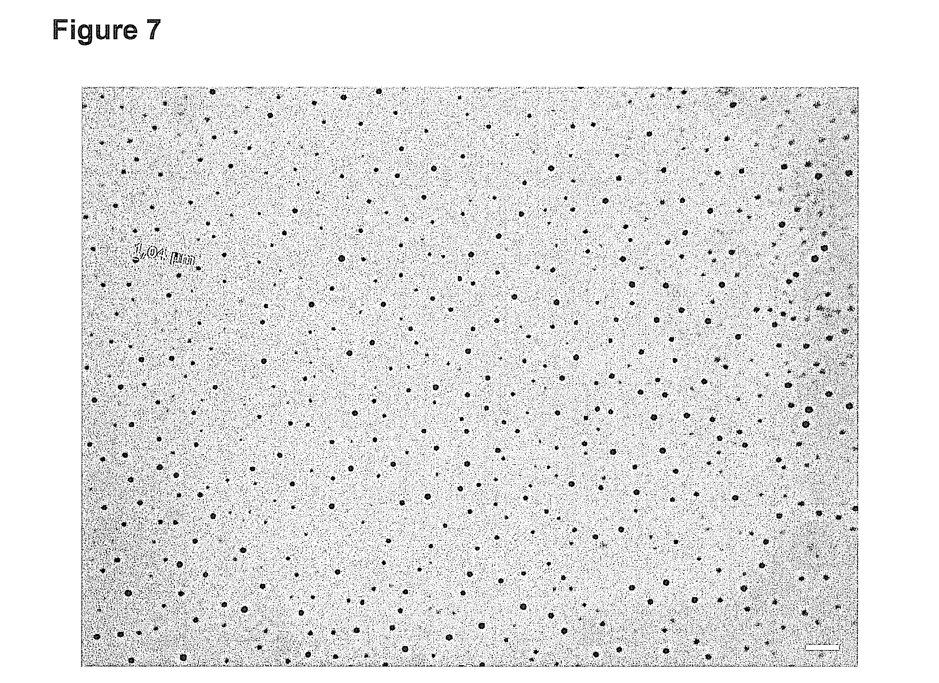

**Figure 7**: Spherical shape of levan particles isolated from *Gluconobacter sp.* TMW 2.1191 in aqueous solution. A solution of 1 g/L was microscopically observed at thousandfold magnification. Scale bar = 5 µm

### EXAMPLES

In general, experiments were conducted under sterile conditions.

For implementation of this invention, the following water kefir isolates were selected:
1. *Gluconobacter sp.* TMW 2.1191, which is a constitutive levansucrase producer, the inducer being sucrose, (Gulitz et al., 2011; Jakob et al., 2012); and
2. *Leuconostoc mesenteroides* TMW 2.1073, which is an inducible dextransucrase producer, the inducer being sucrose (Gulitz et al., 2011; Naessens et al., 2005).

### Example 1: Production of fructan

The water kefir isolate *Gluconobacter sp.* TMW 2.1191 was utilized.

The strain was pre-cultivated aerobically (200 rpm, 30 °C) in a 2 L Erlenmeyer flask supplemented with 200 mL liquid Na-gluconate medium (20 g/L Na-gluconate; 3 g/L yeast extract; 2 g/L peptone from casein; 10 g/L mannitol; 3 g/L glucose; 3 g/L glycerol) to a final OD₆₀₀ = 2.5.

Cells were separated by centrifugation (5000g; 10 min), re-suspended in 200 mL Na-acetate buffer (pH 5.0; 0.1 M) and centrifuged again (5000g; 10 min).

Cells were re-suspended in 200 mL Na-acetate buffer (pH 5.0; 0.1 M; supplemented with 0.1 M sucrose) and incubated for 3 h (200 rpm; 30 °C) in a 2 L Erlenmeyer flask.

Cells were separated by centrifugation (7000g; 10 min) and discarded.

The supernatant was mixed with 800 mL Na-acetate buffer (pH 5.0; 0,1 M; supplemented with 0.1 M sucrose) and incubated for 24 h without stirring (30 °C).

The solution was dialyzed (MWCO: 3 kDa) against ddH₂O for 48 h.

The solution was freeze-dried.

The dried product was weighed

Fructali yield: 4 g (= 20 g/L cell culture)
average gyration radius R_{G} of 428 nm (as measured by AF4-MALS-RI);
average molecular weight M_{w} of 8.26 x 10⁹ Da (as measured by AF4-MALS-RI);

### Example 2: Production of glucan

The water kefir isolate *Leuconostoc mesenteroides* TMW 2.1073 was utilized.

The strain was pre-cultivated aerobically (150 rpm, 30 °C) in a 100 mL Erlenmeyer flask supplemented with 20 mL liquid mMRS medium (de Man et al., 1960) (diluted 1:1 with 0.2 M sucrose) to a final OD₆₀₀ = 0.5.

Cells were separated by centrifugation (5000g; 10 min), re-suspended in 20 mL Na-acetate buffer (pH 5.0; 0.1 M) and centrifuged again (5000g; 10 min).

Cells were re-suspended in 20 mL Na-acetate buffer (pH 5.0; 0.1 M; supplemented with 0.1 M sucrose and 0.05% CaCl₂) and incubated for 2 h (150 rpm; 30 °C) in a 50 mL Sarstedt tube.

Cells were separated by centrifugation (7000g; 10 min) and discarded.

The supernatant was mixed with 160 mL Na-acetate buffer (pH 5.0; 0,1 M; supplemented with 0.1 M sucrose and 0.05% CaCl₂) and incubated for 24 h without stirring (30 °C).

The solution was dialyzed (MWCO: 3 kDa) against ddH₂O for 48 h.

The solution was freeze-dried.

The dried product was weighed.
Glucan yield: 0.7 g (= 35 g/L cell culture)

The features disclosed in the foregoing description, in the claims and/or in the accompanying drawings may, both separately and in any combination thereof, be material for realizing the invention in diverse forms thereof.

### REFERENCES

De Belder, A.N (2003). Dextran. In: Handbook from Amersham Biosciences, 18-1166-12., p. 23.
De Man, J. C., Rogosa, M., & Sharpe, M.E. (1960). A medium for the cultivation of lactobacilli. Journal of Bacteriology, 23, 130.
Donot, F., Fontana, A., Baccou, J.C., & Schorr-Galindo, S. (2012). Microbial exopolysaccharides: Main examples of synthesis, excretion, genetics and extraction. Carbohydrate Polymers, 87, 951-962.
Freitas, F., Alves, V.D., & Reis, M.A. (2011) Advances in bacterial exopolysaccharides: from production to biotechnological applications. Trends in Biotechnology. 29, 388-398.
Goldman, D., Lavid, N., Schwartz, A., Shoham, D., Danino, D., & Shoham, Y. (2008). Two active forms of Zymomonas mobilis levansucrase. An ordered microfibril structure of the enzyme promotes levan polymerization. The Journal of Biological Chemistry, 283, 32209-32217.
Gulitz, A., Stadie, J., Wenning, M., Ehrmann, M.A., & Vogel, R.F. (2011). The microbial diversity of water kefir. International Journal of Food Microbiology, 151, 284-288.
Jakob, F., Meissner, D., & Vogel, R.F. (2012). Comparison of novel GH 68 levansucrases of levan-overproducing Gluconobacter species. Acetic Acid Bacteria*,* doi:10.4081/aab.2012.e2.
Jakob, F., Pfaff, A., Novoa-Carballal, R., Riibsam, H., Becker, T., & Vogel, R.F. (2013). Structural analysis of fructans produced by acetic acid bacteria reveals a relation to hydrocolloid function. Carbohydrate Polymers, 92, 1234-1242.
Korakli, M., & Vogel R.F. (2006). Structure/function relationship of homopolysaccharide producing glycansucrases and therapeutic potential of their synthesised glycans. Applied Microbiology and Biotechnology, 71, 790-803.
Lakshmi Bhavani, A., & Nisha, J. (2010). Dextran - the polysaccharide with versatile uses. International Journal of Pharma and Biosciences, 1(4)*.*
Naessens, M., Cerdobbel, M., Soetaert, W., & Vandamme, E.J. (2005). Leuconostoc dextransucrase and dextran: production, properties and applications. Journal of Chemical Technology and Biotechnology, 80, 845-860.
Robyt, J.F., & Walseth, T.F. (1979). Production, purification, and properties of dextransucrase from Leuconostoc mesenteroides NRRL B-512F*. Carbohydrate Research, 68, 95-111.
Shukla, R., Shulda, S., Bivolarski, V., Iliev, I., Ivanova, I., & Goyal, A. (2011). Structural characterization of insoluble dextran produced by Leuconostoc mesenteroides NRRL B-1149 in the presence of maltose. Food Technology and Biotechnology, 49, 291-296.
Tanaka, M., Murakami, S., Shinke, R., & Aoki, K. (2000). Genetic characteristics of cellulose-forming acetic acid bacteria identified phenotypically as Gluconacetobacter xylinus. Bioscience, Biotechnology, and Biochemistry, 64, 757-760.
Velazquez-Hemandez, M.L., Baizabal-Aguirre, V.M., Bravo-Patin, A., Cajero-Jua, M., Chavez-Moctezuma, M.P., & Valdez-Alarco, J.J. (2009). Microbial fructosyltransferases and the role of fructans. Journal of Applied Microbiology, 106, 1763-1778.

## Claims

1. A method for producing exopolysaccharide(s) (EPS),
comprising the steps of
(a) pre-cultivating cells of a microorganism, that is capable of producing EPS, in a nutrient medium,
(b) separating the cells from the nutrient medium, optionally washing the cells;
(c) re-suspending the cells from step (b) in a solution comprising the inducer(s) for secretion of EPS forming enzymes and, optionally, further inducing compound(s),
and incubating the cells in the solution, whereby the cells excrete the enzyme(s) for EPS formation into the solution,
(d) separating the cells from the solution;
(e) adding the supernatant of (d) to a second solution comprising the substrate(s) for EPS formation and, optionally, further cofactor(s),
and incubating, whereby the EPS is formed;
(f) obtaining the EPS.

2. The method of claim 1, wherein the nutrient medium in step (a) does not contain the inducer(s) for secretion of EPS forming enzymes, preferably when the microorganism is a constitutive producer of the enzyme(s) for EPS formation;
or wherein the nutrient medium in step (a) does contain the inducer(s) for secretion of EPS forming enzymes, preferably when the microorganism is an inducible producer of the enzyme(s) for EPS formation.

3. The method of any of claims 1 or 2, wherein in step (a) the cells are pre-cultivated to the exponential or stationary growth phase.

4. The method of any of claims 1 to 3, wherein the exopolysaccharides (EPS) are homopolysaccharides, such as fructan(s), glucan(s) such as levan, inulin, dextran, cellulose, mutan, alternan, reuteran, or mixtures thereof.

5. The method of any of claims 1 to 4, wherein the microorganism, that is capable of producing EPS, is selected from bacteria or fungi,
and/or
wherein the microorganism is a genetically engineered strain that recombinantly produces EPS forming enzymes.

6. The method of any of the preceding claims, wherein the inducer(s) for secretion of EPS forming enzymes and/or the substrate(s) for EPS formation is(are) selected from glucose, sucrose, fructose, galactose, trehalose, mannose, melobiose, raffinose, maltotriose, maltotetraose, maltose, lactose, lactulose, methyl-β-galactopyranoside, methyl-α-galactopyranoside, cellobiose, gentobiose, methyl-β-D-glucopyranoside, methyl-α-D-glucopyranoside, esculin, ribose, arabinose, xylose, palatinose, rhamnose, fucose, melezitose, D(+) arabitol, L(-) arabitol, xylitol, dulcitol, tagatose, glycerol, myoinositol, mannitol, maltitol, turanose, sorbitol, adonitol, lyxose, erythritol, hydrolyzed starch, starch hydrolyzates, mixtures and combinations of these sugars, and mixtures comprising at least one of these sugars,
and/or
wherein the inducer(s) for secretion of EPS forming enzymes and/or the substrate(s) for EPS formation is(are) EPS, such as glucooligosaccharides, glucans, fructooligosaccharides, fructans

7. The method of any of the preceding claims, wherein the solutions in step (c) and/or in step (e) furthermore contain salts, bases, and acids or combinations thereof.

8. The method of any of the preceding claims, wherein the EPS are obtained or isolated in step (f) via filtration, (ultra)centrifugation, ion exchange and/or dialysis,
wherein obtaining the EPS in step (f) preferably does not require precipitation.

9. The method of any of the preceding claims, wherein EPS is directly isolated from the the solution of step (c), in a case where the inducer(s) for secretion of EPS forming enzymes and the substrate(s) of the EPS forming enzyme are identical.

10. The method of any of the preceding claims, further comprising stabilizing the obtained or isolated EPS of step (f) via lyophilization or other suitable preparation methods,
and/or
further comprising a heat deactivation of the residual proteins, such as enzymes.

11. The method of any of the preceding claims, wherein the yield of EPS is in the range from about 2 g to about 1000 g per litre cell culture.

12. An exopolysaccharide (EPS) obtained by the method according to any of claims 1 to 11,
preferably being a homopolysaccharide, such as fructan, glucan, e.g. levan, inulin, dextran, mutan, alternan, reuteran, or mixtures thereof.

13. Use of an exopolysaccharide (EPS) obtained by the method according to any of claims 1 to 11 or of an exopolysaccharide (EPS) according to claim 12 in
food industry,
such as
as thickening agent, stabilizing agent, emulsifying agent, gelating agent,
as hydro-colloids,
as inclusion bodies (e.g. for the controlled release of vitamins, aroma compounds)
pharmaceutics,
such as for drug delivery, e.g. with spherical inclusion bodies,
biological degradable synthetics
cosmetic industry
such as
as bioadhesive substances,
as thickening agent, stabilizing agent, emulsifying agent, gelating agent, (e.g. in tooth paste, lipstick, creames)
biomedicine or environment engineering,
such as
in (artificial) biofilms
blood plasma volume expanders
chemical industry
such as
as molecular weight standard
as initiator molecules for further processing (derivatized polymers)
as column materials for chromatographic techniques.
paper/materials/polymer industry
such as
as surface smoothener
as structural improver
as firmness improver
as water binding agent
as compatibility enhancer (e.g. in implants).

14. An enzyme or enzymes obtained during and/or after step (c), after step (d) and/or during and/or after the incubation of step (e) of the method according to any of claims 1 to 9, preferably being glycosyl- and/or fructosyl-transferase(s) belonging to the glycoside hydrolase families 70 and 68.

15. Use of the enzyme(s) of claim 14 for producing EPS, or in the food industry, pharmaceutics, cosmetic industry, biomedicine or environmental engineering, chemical industry, paper/materials/polymer industry.
